Europäisches Patentamt

European Patent Office

Office européen des brevets

(19)

(11) Veröffentlichungsnummer : **0 033 392**
**B1**

(12) **EUROPÄISCHE PATENTSCHRIFT**

(45) Veröffentlichungstag der Patentschrift :
30.03.83

(21) Anmeldenummer : 80108170.4

(22) Anmeldetag : 23.12.80

(51) Int. Cl.³ : **C 07 C102/06, C 07 C103/46,
C 07 C149/247, C 07 D209/20**

(54) Verfahren zur Acylierung von Aminocarbonsäuren.

(30) Priorität : 02.02.80 DE 3003898

(43) Veröffentlichungstag der Anmeldung :
12.08.81 Patentblatt 81/32

(45) Bekanntmachung des Hinweises auf die Patenterteilung : 30.03.83 Patentblatt 83/13

(84) Benannte Vertragsstaaten :
AT BE CH DE FR GB IT LI LU NL SE

(56) Entgegenhaltungen :
FR A 926 115
FR A 2 375 189

(73) Patentinhaber : DYNAMIT NOBEL AKTIENGESELL-SCHAFT
Patentabteilung Postfach 1209
D-5210 Troisdorf, Bez. Köln (DE)

(72) Erfinder : Giselher, Franzmann, Dr.
In der Schlade 61
D-5810 Witten (DE)

Jouve, 18, rue St-Denis, 75001 Paris, France

# Verfahren zur Acylierung von Aminocarbonsäuren

Die Erfindung betrifft ein einfaches und kostengünstiges Verfahren zur Acylierung, besonders zur Acetylierung von Aminocarbonsäuren. Besonders teure Aminosäuren, wie z. B. Phenylalanin und Tryptophan können so vorteilhaft acyliert werden.

Es ist bekannt, mit Hilfe von Carbonsäureanhydriden oder -halogeniden Aminocarbonsäuren zu acylieren. Die bekannten Verfahren ergeben entweder keine hohen Ausbeuten oder keine hohe Reinheit der Produkte. Insbesondere die Acetylierung von Phenylalanin mit Acetanhydrid ergibt ein unreines Produkt. Der stets erforderliche Überschuß an Acetanhydrid wird umso höher, je mehr man eine befriedigende Reinheit von N-Acetylphenylalanin anstrebt. Die bei der Acetylierung mit Acetanhydrid anfallende Essigsäure behindert die Isolierung der Produkte und verhindert hohe Ausbeuten an reinem Produkt.

Gute Ausbeuten und Reinheiten ergibt die Acetylierung von Aminocarbonsäuren mit Keten nach der DE-OS 27 41 081. Keten muß aber in einem Generator jeweils frisch erzeugt und sofort verbraucht werden. Dieses Verfahren ist daher erst bei relativ großen Mengen rentabel.

Es bestand daher die Aufgabe, ein Verfahren zur Acylierung von Aminocarbonsäuren zu finden, welches Produkte hoher Reinheit mit hoher Ausbeute kostengünstig liefert. Es wurde gefunden, daß Aminocarbonsäuren und deren Alkalisalze mit Carbonsäureniedrigalkylestern in Gegenwart von Alkalialkoholaten überraschenderweise quantitativ acyliert werden.

Gegenstand der Erfindung ist daher ein Verfahren zur Acylierung von Aminocarbonsäuren, welches dadurch gekennzeichnet ist, daß Aminocarbonsäuren, deren Alkalisalze oder Erdalkalisalze mit Carbonsäure-niedrigalkylestern der allgemeinen Formel $R_1$—CO—O—$R_2$, worin $R_1$ Wasserstoff oder ein geradkettiger, verzweigter oder cyclischer Kohlenwasserstoffrest mit 1 bis 30 C-Atomen bedeutet, der gegebenenfalls substituiert sein kann und worin $R_2$ ein geradkettiger, verzweigter oder cyclischer Kohlenwasserstoffrest mit 1 bis 8 C-Atomen bedeutet, der gegebenenfalls substituiert sein kann, in Gegenwart von Alkali- oder Erdalkalialkoholaten, umgesetzt werden.

Man erhält Produkte hoher Reinheit von 98 bis 100 % mit hoher Ausbeute von mindestens 90 %, im allgemeinen 95 bis 99 %.

Das Verfahren gestattet auch bei den relativ kleinen Mengen, wie sie z. B. bei Phenylalanin und Tryptophan in Frage kommen, eine wirtschaftliche Herstellung der Acylaminosäuren.

Das erfindungsgemäße Verfahren läßt sich sehr einfach durchführen : die zu acylierende Aminocarbonsäure, das Alkalialkoholat und der Carbonsäureniedrigalkylester werden gemischt und unter Rühren auf Temperaturen von 40 bis 200 °C erhitzt. Nach 2 bis 4 Stunden ist die Reaktion beendet. Überschüssiger Carbonsäureniedrigalkylester und bei der Reaktion aus dem Alkalialkoholat sowie aus dem Ester freigesetzter Alkohol können abdestilliert werden. Der Rückstand wird nach gängigen Verfahren aufgearbeitet. Es wird beispielsweise mit Wasser versetzt und mit einer Säure die N-Acetylaminosäure ausgefällt oder die Alkalisalze der N-Acylaminosäuren gewonnen.

Die Zusammensetzung des Reaktionsgemisches kann in weiten Grenzen variiert werden. Setzt man eine einbasische Aminocarbonsäure ein, so benötigt man 1 Mol Alkalialkoholat pro Mol Aminosäure zur Bildung des Alkalisalzes. Man kann jedoch auch die entsprechenden Alkalisalze der Aminocarbonsäuren einsetzen. Über die äquimolare Menge hinaus benötigt man einen Überschuß an Alkalialkoholat als Katalysator für die Acylierung. Da die eingesetzten Produkte häufig Wasserspuren enthalten, wählt man den Überschuß an Alkalialkoholat nicht zu knapp. So setzt man pro Mol Aminocarbonsäure insgesamt vorzugsweise 1,2 bis 2,0 Mol, sehr bevorzugt 1,4 bis 1,6 Mol, Alkalialkoholat zur wasserfreien Salzbildung und als Katalysator ein. Unter ca. 1,2 Mol Alkoholat pro Mol Aminosäure werden die Ausbeuten schlechter, wenn man Wasserspuren nicht sehr sorgfältig ausschließt. Mehr als 2 Mol Alkoholat pro Mol Aminocarbonsäure einzusetzen, ist in der Regel unnötig. Im Falle der Verwendung von Alkalisalzen wird die Menge des Alkoholats um die schon vorhandene Alkalimenge vermindert, im Falle mehrbasischer Aminocarbonsäuren um die zur zusätzlichen Salzbildung erforderliche Menge Alkoholat erhöht.

Der Carbonsäure-niedrigalkylester soll mindestens in 1 : 1 molarem Verhältnis, bezogen auf die zu acylierenden Aminogruppen, eingesetzt werden. Meistens setzt man einen Überschuß von 50 bis 500 Mol % ein, vor allem, um bei der Reaktion ein rührbares Gemisch, im Idealfall eine klare Lösung, zu erhalten. In vielen Fällen ist es vorteilhaft, zu den drei Komponenten Aminocarbonsäure, Alkoholat und Carbonsäureester noch ein Lösemittel oder einen Lösungsvermittler zuzusetzen. Man verwendet z. B. einen Alkohol und kann damit die erforderliche Menge des Carbonsäureesters vermindern. Naheliegend ist in diesem Fall, den gleichen Alkohol zu verwenden, der bei der Reaktion aus dem Alkalialkoholat bzw. dem Carbonsäureniedrigalkylester freigesetzt wird. Die Alkylreste von Alkoholat, Ester und eventuell zugesetztem Alkohol können jedoch im Prinzip beliebig, d. h. gleich oder verschieden, gewählt werden.

Die Reaktionstemperatur liegt vorzugsweise bei 50 bis 100 °C, sehr bevorzugt beim Siedepunkt des jeweiligen Reaktionsgemisches, kann jedoch im Bereich ab ca. 40 °C in weiten Grenzen nach oben variiert werden. Temperaturen unter ca. 40 °C sind weniger sinnvoll, da die Reaktionsgeschwindigkeit kleiner wird. Höhere Temperaturen erleichtern das Rühren der Mischung bzw. die Bildung klarer Lösungen.

Ist die Acylierung optisch aktiver Aminosäuren ohne Racemisierung beabsichtigt, so empfiehlt es sich, die Reaktionstemperatur möglichst tief, gleichzeitig aber auch die Reaktionsdauer möglichst kurz zu halten.

Erhöhter Druck bis 50 bar ist möglich, aber ohne Einfluß auf das Reaktionsergebnis. Das Verfahren wird daher vorzugsweise bei Normaldruck ausgeführt.

Als Aminocarbonsäuren werden beliebige, eine oder mehrere, primäre oder sekundäre, bevorzugt aliphatische Aminogruppen enthaltende ein- oder mehrbasige Carbonsäuren verstanden. Aus Gründen des Bedarfs sind von diesen die α-Aminocarbonsäuren von der Struktur der natürlichen Aminosäuren bevorzugt. Die Aminocarbonsäuren können synthetischer oder natürlicher Herkunft sein.

Solche Aminosäuren sind z. B. Alanin, Valin, Methionin, Serin, Phenylalanin, Tyrosin, Tryptophan, 3,4-Dihydroxyphenylalanin (DOPA).

Als Carbonsäure-niedrigalkylester sind die der gesättigten geradkettigen Monocarbonsäuren mit 1 bis 18, insbesondere mit 1 bis 4 Kohlenstoffatomen, ganz besonders die der Essigsäure, unter den Alkylgruppen der Ester besonders die Äthyl- und Methylgruppe, bevorzugt.

Beispiele für solche Carbonsäure-niedrigalkylester sind : Ameisensäureäthylester, Essigsäuremethylester, Essigsäureäthylester, Essigsäureisopropylester, Essigsäurecyclohexylester, Essigsäurebenzylester, Propionsäuremethylester, Buttersäuremethylester, Isobuttersäureäthylester, Benzoesäuremethylester, p-Toluylsäuremethylester, Laurinsäuremethylester, Myristinsäuremethylester, Stearinsäuremethylester, Chloressigsäureäthylester.

Die zur Salzbildung und als Katalysator für das erfindungsgemäße Verfahren einsetzbaren Alkalialkoholate haben die allgemeine Formel $R_2$—O—Me, wobei $R_2$ die obenangegebene Bedeutung hat und Me ein Mol Alkalimetall oder gegebenenfalls ein halbes Mol Erdalkalimetall ist. Beispiele sind : Natriummethylat, Natriumäthylat, Kaliumisopropylat.

Prinzipiell ist nach dem erfindungsgemäßen Verfahren auch der Einsatz von Erdalkalialkoholaten möglich, wegen der schwereren Löslichkeit der Aminosäure- bzw. Acylaminosäure-erdalkalisalze jedoch in den meisten Fällen nicht vorteilhaft.

Als Produkt kann die freie N-Acylaminocarbonsäure oder deren Alkalisalze bzw. gegebenenfalls Erdalkalisalze oder auch die Lösungen der Salze gewonnen werden.

Die freien N-Acylaminosäuren werden mit Mineralsäuren wie Salzsäure oder Schwefelsäure in mindestens äquivalenter Menge gefällt.

Die Salze werden nach Versetzen mit Wasser aus wässriger Lösung gewonnen.

Der Überschuß von Carbonsäure-niedrigalkylester kann nach beendeter Reaktion, eventuell im Gemisch mit Alkohol, abdestilliert und für weitere Ansätze wiederverwendet werden. Gegebenenfalls wird Wasser vor dieser Destillation zugesetzt, damit die Destillation nicht durch ausfallende Salze erschwert wird.

Es ist möglich, Acylaminosäure-alkalisalze rein herzustellen. So kristallisiert z. B. Acetyl-DL-tryptophan-natriumsalz-tetrahydrat aus der Reaktionsmischung aus, wenn man nach Wasserzugabe und Abdestillieren des Alkohol/Ester-Gemisches aus einem Acetylierungsansatz mit Essigsäureniedrigalkylester die wässrig alkalische Lösung neutralisiert.

Leicht wasserlösliche Acylaminosäure-alkalisalze, beispielsweise des Alanins, lassen sich gewinnen, wenn man das Reaktionsgemisch ohne Wasserzugabe eindampft. Entweder trennt man dann das gewünschte Produkt aus einer teilweise eingedampften Lösung ab, die so beschaffen sein muß, daß das Alkalialkoholat gerade noch in Lösung bleibt, oder man dampft zur Trockne ein und gewinnt so ein Gemisch aus Alkalialkoholat und Acylaminosäure-alkalisalz. Durch Zumischen einer dem überschüssigen Alkoholat äquivalenten Menge Acylaminosäure vor oder nach dem Abdestillieren von Alkohol und gegebenenfalls Ester läßt sich reines, wasserfreies Acylaminosäure-alkalisalz gewinnen.

Bei beabsichtigtem Einsatz der hergestellten Acylaminosäuren und -alkalisalzen zur selektiven enzymatischen Verseifung bzw. Entacylierung kann Wasser zugesetzt, Alkohol und gegebenenfalls Ester abdestilliert, die Lösung mit der entsprechenden Acylaminosäure auf den pH-Bereich der enzymatischen Spaltung neutralisiert und nach Verdünnung sehr vorteilhaft direkt eingesetzt werden.

Es ist ein besonderer Vorteil des vorliegenden Verfahrens, daß die Produkte in sehr hoher Ausbeute von 95 bis 99 % und in sehr hoher Reinheit entstehen und gewinnbar sind.

Das Verfahren ist sehr schonend, so daß es auch und ganz besonders zur Acylierung teurer Aminosäuren geeignet ist.

Nach dem Verfahren erfolgt selektiv die Acylierung der Aminogruppe. Die Indolyl-NH-Gruppen beispielsweise des Tryptophan oder die phenolische OH-Gruppe des Tyrosins wird nicht acyliert.

Beispiel 1

49,5 g DL-Phenylalanin (0,3 Mol), 300 Ml Essigsäuremethylester und 24,3 g Natriummethylat (0,45 Mol) werden gemischt und unter Rühren 4 Stunden am Rückfluß gekocht. Überschüssiger Essigsäuremethylester wird abdestilliert, der Rückstand wird in 200 ml Wasser aufgenommen. Mit 75 ml 6 molarer Salzsäure wird N-Acetyl-DL-phenylalanin ausgefällt, nach Kühlen auf 5-10 °C abgesaugt, mit 200 ml Wasser gewaschen und getrocknet.

Ausbeute : 59,0 g (95 %)
Asche : 0,1 %

Säurezahl : 271 (ber. 271)
Fp : 152 °C

Beispiel 2

495 g DL-Phenylalanin (3 Mol), 245 g Natriummethylat (4,5 Mol), 600 ml Essigsäuremethylester und 150 ml Methanol werden gemischt und unter Rühren 4 Stunden am Rückfluß gekocht. Nach Zusatz von 500 ml Wasser wird überschüssiger Essigsäuremethylester im Gemisch mit Methanol und Wasser abdestilliert (580 ml). Die Lösung wird mit 1 700 ml Wasser verdünnt. Das N-Acetyl-DL-phenylalanin wird mit 138 ml konzentrierter Schwefelsäure ausgefällt, nach Kühlen auf 10 °C abgesaugt, zunächst mit 2 l und nochmals mit 1,3 l Wasser gewaschen und getrocknet.
Ausbeute : 582 g (94 %)
Asche : < 0,1 %
Säurezahl : 272 (ber. 271)
Fp : 154 °C

Beispiel 3

Entsprechend Beispiel 2 wird anstelle von DL-Phenylalanin als Aminosäure DL-Leucin mit entsprechendem Ergebnis acyliert.

Beispiel 4

Beispiel 2 wird wiederholt. Nach der Reaktion wird bei der Wasserzugabe das zweite Waschwasser eines vorhergehenden Ansatzes mitverwendet.
Ausbeute : 608 g (98 %)
Asche : 0,05 %
Säurezahl : 271
Fp : 154-155 °C

Beispiel 5

49,5 g L-Phenylalanin, 24,3 g Natriummethylat, 60 ml Essigsäuremethylester und 15 ml Methanol werden gemischt und unter Rühren 2 Stunden am Rückfluß gekocht. Nach Zugabe von 50 ml Wasser wird überschüssiger Essigsäuremethylester im Gemisch mit Methanol und Wasser abdestilliert (70 ml). Der Rückstand wird mit 50 ml Wasser verdünnt. Mit 40 ml konzentrierter Salzsäure (12 molar) wird N-Acetyl-L-phenylalanin ausgefällt, nach Kühlen auf 5-10 °C abgesaugt, mit 300 ml Wasser gewaschen und getrocknet.
Ausbeute : 57,5 g (93 %)
Asche : < 0,1 %
Säurezahl : 274
Chlorid : 0,17 %
spezifischer Drehwert (5 % in Äthanol) :
$\alpha_D^{25} = + 47,4°$ ($\hat{=}$ 93 % optische Reinheit)

Beispiel 6

49,5 g DL-Phenylalanin, 24,3 g Natriummethylat, 80 ml Essigsäureäthylester und 20 ml Methanol werden gemischt und unter Rühren 4 Stunden am Rückfluß gekocht. Nach Zugabe von 50 ml Wasser wird überschüssiger Essigsäureäthylester im Gemisch mit Methanol, Äthanol und Wasser abdestilliert (100 ml). Der Rückstand wird mit 250 ml Wasser verdünnt. Mit 39 ml konzentrierter Salzsäure wird N-Acetyl-DL-phenylalanin ausgefällt, nach Kühlen auf 5-10 °C abgesaugt, mit 200 ml Wasser gewaschen und getrocknet.
Ausbeute : 59,7 g (96 %)
Asche : < 0,1 %
Chlorid : 0,04 %
Säurezahl : 270
Fp : 154 °C

Beispiel 7

Beispiel 6 wird wiederholt, jedoch wird statt Essigsäureäthylester Propionsäuremethylester verwendet. Produkt : N-Propionyl-DL-phenylalanin.
Ausbeute : 63,5 g (96 %)
Asche : < 0,1 %
Chlorid : 0,09 %
Säurezahl : 252 (ber. 254)
Fp : 133 °C

Beispiel 8

61,3 g DL-Tryptophan (0,3 Mol), 24,3 g Natriummethylat (0,45 Mol), 80 ml Essigsäuremethylester und 20 ml Methanol werden gemischt und unter Rühren 4 Stunden am Rückfluß gekocht. Nach Zugabe von 50 ml Wasser werden 60 ml Ester/Alkohol/Wasser-Gemisch abdestilliert. Der Rückstand wird mit 50 ml Wasser verdünnt. Mit 38 ml konzentrierter Salzsäure wird $N_\alpha$-Acetyl-DL-tryptophan ausgefällt, nach Kühlen auf 10 °C abgesaugt, mit 300 ml Wasser gewaschen und getrocknet.
Ausbeute : 70,2 g (95 %)
Asche : < 0,1 %
Chlorid : 0,3 %
Säurezahl : 230 (ber. 228)
Fp : 203 °C

Beispiel 9

Beispiel 8 wird wiederholt. Statt mit Salzsäure auszufällen, wird zunächst mit 2 ml konzentrierter Salzsäure auf pH 7 neutralisiert. Nach Kühlen auf 10 °C wird $N_\alpha$-Acetyl-DL-tryptophannatriumsalz-tetrahydrat abgesaugt, mit 20 ml Wasser gewaschen und schonend getrocknet. Man erhält 55,8 g $N_\alpha$-Acetyl-DL-tryptophannatriumsalz-tetrahydrat, entsprechend 40,4 g $N_\alpha$-Acetyl-DL-tryptophan.
Aus Mutterlauge und Waschfiltrat wird mit 15,7 ml konzentrierter Salzsäure $N_\alpha$-Acetyl-DL-tryptophan ausgefällt. Man erhält 29,7 g.
Gesamtausbeute : 95 %

Beispiel 10

44,7 g DL-Methionin (0,3 Mol), 24,3 g Natriummethylat, 60 ml Essigsäuremethylester und 15 ml Methanol werden gemischt und unter Rühren 4 Stunden am Rückfluß gekocht. Nach Zusatz von 50 ml Wasser wird überschüssiger Essigsäuremethylester im Gemisch mit Methanol und Wasser abdestilliert (60 ml). Aus dem Rückstand wird mit 78 ml halb-konzentrierter Salzsäure (6 Mol) N-Acetyl-DL-methionin ausgefällt, nach Kühlen auf 5 °C abgesaugt, mit 100 ml Wasser (10 °C) gewaschen und getrocknet. Aus der Mutterlauge wird durch Extraktion mit Essigsäureäthylester weiteres Produkt gewonnen und mit dem gefällten Produkt vereinigt.

Ausbeute : 51,6 g (90 %)

Asche : 0 %

Chlorid : 0,09 %

Säurezahl : 293 (ber. 294)

Fp : 115 °C

Beispiel 11

Beispiel 6 wird wiederholt. Statt 80 ml Essigsäureäthylester werden 100 g Laurinsäuremethylester verwendet. Es bildet sich in guter Ausbeute N-Lauryl-DL-phenylalanin.

**Ansprüche**

1. Verfahren zur Acylierung von Aminocarbonsäuren, dadurch gekennzeichnet, daß Aminocarbonsäuren, deren Alkalisalze oder Erdalkalisalze mit Carbonsäure-niedrigalkylestern der allgemeinen Formel R₁—CO—O—R₂, worin R₁ Wasserstoff oder ein geradkettiger, verzweigter oder cyclischer Kohlenwasserstoffrest mit 1 bis 30 C-Atomen bedeutet, der gegebenenfalls substituiert sein kann und worin R₂ ein geradkettiger, verzweigter oder cyclischer Kohlenwasserstoffrest mit 1 bis 8 C-Atomen bedeutet, der gegebenenfalls substituiert sein kann, in Gegenwart von Alkali- oder Erdalkalialkoholaten umgesetzt werden.

2. Verfahren nach Anspruch 1, dadurch gekennzeichnet, daß die Reaktion bei 40 bis 200 °C durchgeführt wird.

3. Verfahren nach Anspruch 2, dadurch gekennzeichnet, daß die Reaktion bei 50 bis 100 °C durchgeführt wird.

4. Verfahren nach einem der Ansprüche 1 bis 3, dadurch gekennzeichnet, daß 1,2 bis 2 Mol Alkalialkoholat, bezogen auf 1 Mol Aminocarbonsäure, verwendet werden.

5. Verfahren nach einem der Ansprüche 1 bis 4, dadurch gekennzeichnet, daß 1 bis 5 Mol Carbonsäure-niederalkylester je Mol Aminocarbonsäure verwendet werden.

6. Verfahren nach einem der Ansprüche 1 bis 5, dadurch gekennzeichnet, daß als Alkalialkoholat ein Natriumalkoholat eingesetzt wird.

7. Verfahren nach Anspruch 6, dadurch gekennzeichnet, daß als Natriumalkoholat Natriummethylat eingesetzt wird.

8. Verfahren nach einem der Ansprüche 1 bis 5, dadurch gekennzeichnet, daß als Carbonsäureniedrigalkylester ein Essigsäure-niedrigalkylester

eingesetzt wird.

9. Verfahren nach Anspruch 8, dadurch gekennzeichnet, daß als Carbonsäure-niedrigalkylester Essigsäuremethylester eingesetzt wird.

10. Verfahren nach einem der Ansprüche 1 bis 9, dadurch gekennzeichnet, daß dem Reaktionsgemisch ein Lösemittel oder ein Lösungsvermittler, vorzugsweise ein Alkohol, besonders bevorzugt Methanol, zugesetzt wird.

11. Verfahren nach einem der Ansprüche 1 bis 10, dadurch gekennzeichnet, daß synthetische oder natürliche α-Aminocarbonsäuren acyliert werden.

12. Verfahren nach Anspruch 11, dadurch gekennzeichnet, daß Phenylalanin, Tryptophan oder Methionin, sehr bevorzugt Phenylalanin, acyliert werden.

13. Verfahren nach einem der Ansprüche 1 bis 12, dadurch gekennzeichnet, daß nach der Umsetzung die N-Acylaminosäure als Alkalisalz als oder freie N-Acylaminosäure gewonnen wird.

**Claims**

1. Process for the acylation of aminocarboxylic acids, characterised in that aminocarboxylic acids are reacted as their alkali salts or alkaline earth salts in the presence of the alkali or alkaline earth alcoholates with carboxylic acid-lower alkyl esters of the general formula R₁—CO—O—R₂, wherein R₁ denotes hydrogen or a straight chained, branched or cyclic hydrocarbon residue with 1 to 30 C-atoms, which can optionally be substituted and wherein R₂ denotes a straight chained, branched or cyclic hydrocarbon residue with 1 to 8 C-atoms which can optionally be substituted.

2. Process according to claim 1, characterised in that the reaction is carried out at 40 to 200 °C.

3. Process according to claim 2, characterised in that the reaction is carried out at 50 to 100 °C.

4. Process according to one of claims 1 to 3, characterised in that 1.2 to 2 mol of alkali alcoholate, related to 1 mol of aminocarboxylic acid, are used.

5. Process according to one of claims 1 to 4, characterised in that 1 to 5 mol of carboxylic acid-lower alkyl ester are used per mol of aminocarboxylic acid.

6. Process according to one of claims 1 to 5, characterised in that a sodium alcoholate is employed as alkali alcoholate.

7. Process according to claim 6, characterised in that sodium methylate is employed as sodium alcoholate.

8. Process according to one of claims 1 to 5, characterised in that an acetic acid lower alkyl ester is employed as carboxylic acid-lower alkyl ester.

9. Process according to claim 8, characterised in that acetic acid methyl ester is employed as carboxylic acid lower alkyl ester.

10. Process according to one of claims 1 to 9, characterised in that a solvent or a solubilizer, preferably an alcohol, especially preferably methanol, is added to the reaction mixture.

11. Process according to one of claims 1 to 10, characterised in that synthetic or natural α-aminocarboxylic acids are acylated.

12. Process according to claim 11, characterised in that phenylalanine, tryptophane or methionine, very preferably phenylalanine, are acylated.

13. Process according to one of claims 1 to 12 characterised in that, after the reaction, the N-acylamino acid is recovered as alkali salt or as free N-acylamino acid.

## Revendications

1. Procédé d'acylation d'acides aminés, caractérisé en ce qu'on fait réagir des acides aminés, leurs sels alcalins ou leurs sels alcalinoterreux, avec des esters d'alkyle inférieur d'acides carboxyliques de formule générale $R_1$—CO—O—$R_2$, où $R_1$ représente l'hydrogène ou un groupe hydrocarboné à chaîne droite, ramifiée ou cyclique, ayant de 1 à 30 atomes de carbone, qui peut être éventuellement substitué, et où $R_2$ représente un groupe hydrocarboné à chaîne droite, ramifiée ou cyclique ayant de 1 à 8 atomes de carbone, qui peut être éventuellement substitué, en présence d'alcoolates alcalins ou alcalinoterreux.

2. Procédé selon la revendication 1, caractérisé en ce que la réaction est conduite entre 40 et 200 °C.

3. Procédé selon la revendication 2, caractérisé en ce que la réaction est conduite entre 50 et 100 °C.

4. Procédé selon l'une quelconque des revendications 1 à 3, caractérisé en ce qu'on utilise de 1,2 à 2 moles d'alcoolate alcalin par mole d'acide aminé.

5. Procédé selon l'une quelconque des revendications 1 à 4, caractérisé en ce qu'on utilise de 1 à 5 moles d'ester d'alkyle inférieur d'acide carboxylique par mole d'acide aminé.

6. Procédé selon l'une quelconque des revendications 1 à 5, caractérisé en ce qu'on utilise comme alcoolate alcalin un alcoolate de sodium.

7. Procédé selon la revendication 6, caractérisé en ce qu'on utilise comme alcoolate de sodium l'éthylate de sodium.

8. Procédé selon l'une quelconque des revendications 1 à 5, caractérisé en ce qu'on utilise comme ester d'alkyle inférieur d'acide carboxylique un acétate d'alkyle inférieur.

9. Procédé selon la revendication 8, caractérisé en ce qu'on utilise comme ester d'alkyle inférieur d'acide carboxylique l'acétate de méthyle.

10. Procédé selon l'une quelconque des revendications 1 à 9, caractérisé en ce qu'on ajoute au mélange réactionnel un solvant ou un médiateur de dissolution, de préférence un alcool, et tout particulièrement le méthanol.

11. Procédé selon l'une quelconque des revendications 1 à 10, caractérisé en ce qu'on acyle des acides α-aminés synthétiques ou naturels.

12. Procédé selon la revendication 11, caractérisé en ce qu'on acyle la phénylalanine, le tryptophane ou la méthionine, et de préférence la phénylalanine.

13. Procédé selon l'une quelconque des revendications 1 à 12, caractérisé en ce que, selon la réaction, on obtient l'acide N-acylaminé sous forme d'un sel alcalin ou d'un acide N-acylaminé libre.